(19) Europäisches Patentamt / European Patent Office / Office européen des brevets

(11) **EP 4 032 535 A1**

(12) **EUROPEAN PATENT APPLICATION**
published in accordance with Art. 153(4) EPC

(43) Date of publication:
**27.07.2022 Bulletin 2022/30**

(21) Application number: **20864928.5**

(22) Date of filing: **18.09.2020**

(51) International Patent Classification (IPC):
**A61K 31/726** (2006.01)     **A61P 11/02** (2006.01)

(52) Cooperative Patent Classification (CPC):
**A61K 31/726; A61P 11/02**

(86) International application number:
**PCT/JP2020/035438**

(87) International publication number:
**WO 2021/054440 (25.03.2021 Gazette 2021/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.09.2019  JP 2019170955**

(71) Applicants:
• **University of Fukui**
**Fukui-shi, Fukui 910-8507 (JP)**
• **Maruho Co., Ltd.**
**Osaka 531-0071 (JP)**

(72) Inventors:
• **FUJIEDA Shigeharu**
**Yoshida-gun, Fukui 910-1193 (JP)**
• **TAKABAYASHI Tetsuji**
**Yoshida-gun, Fukui 910-1193 (JP)**
• **YOSHIDA Kanako**
**Yoshida-gun, Fukui 910-1193 (JP)**
• **WATANABE Hideki**
**Osaka-shi, Osaka 531-0071 (JP)**
• **FUJIKAWA Koki**
**Osaka-shi, Osaka 531-0071 (JP)**

(74) Representative: **Mewburn Ellis LLP**
**Aurora Building**
**Counterslip**
**Bristol BS1 6BX (GB)**

(54) **PHARMACEUTICAL COMPOSITION**

(57)     The present invention relates to a nasal polyps reducing agent containing a polysaccharide selected from a polysulfated chondroitin sulfate, chondroitin sulfate, dermatan sulfate, keratan sulfate, heparan sulfate, dextran sulfate, pentosan polysulfate (PPS), chondroitin, glucomannan, inulin and xylo-oligosaccharide, or a salt thereof as an active ingredient, a pharmaceutical composition, a method for reducing nasal polyps or a method for preventing/treating nasal polyps. According to the present invention, it is possible to provide an effective and safe nasal polyps reducing agent.

[Figure 1]

**Description**

Technical Field

The present invention relates to a pharmaceutical composition containing a predetermined polysaccharide used as a nasal polyps reducing agent, a method for treating nasal polyps using a predetermined polysaccharide, or others.

Background Art

Nasal polyps are fleshy outgrowths of the nasal mucosa that form at the site (usually around the ostia of the maxillary sinuses) of dependent edema in the lamina propria of the mucous membrane. Nasal polyps are a major nose disorder that occurs sometimes in association with chronic sinusitis and worsens nasal congestion of chronic sinusitis (MSD manual professional edition, https://www.msdmanuals.com/ja-jp).

Various causes of nasal polyps are pointed out including edema due to elevation of vascular permeability, prolapse of the lamina propria and accumulation of extracellular matrix. In addition, involvement of various cytokines and growth factors in nasal polyps have been reported. As the growth factor involved in nasal polyps, vascular endothelial growth factor and platelet-derived growth factor are reported (Non Patent Literature 1: Kawasaki Medical School Journal 35 (1): 39-50, 2009).

Chronic sinusitis is defined as a disease characterized by respiratory symptoms such as nasal congestion, rhinorrhea, post-nasal drip and cough that persist for 3 months or more. The causes for sinusitis include viral, bacterial, mycotic, allergic or eosinophilic factors (disease state is unknown). Other than these, factors such as morphologic difference of the nasal cavity, living environment and heredity influence each other to develop a complicated disease state including formation of nasal polyps (Non Patent Literature 2: Bulletin of the Japan Otolaryngology Society, 2018, 121, 1118-1120).

Of the cases of chronic sinusitis, chronic sinusitis associated with nasal polyps includes eosinophilic sinusitis and non-eosinophilic sinusitis. Of them, eosinophilic sinusitis is a designated intractable disease, more specifically, refractory sinusitis developed in adults. Eosinophilic sinusitis exhibits severe nasal congestion and olfactory dysfunction due to multiple nasal polyps formed in both cavities and thick nasal discharge. Antibacterial drugs are ineffective for treating the disease and the disease responds only to steroid oral intake. Nasal polyps filled the nasal cavity are surgically excised out from the nasal cavity (nasal sinus); however, nasal polyps immediately regenerate. Regeneration of nasal polyps is a problem.

It has been reported that nasal polyps are treated by a drug such as a steroid topically applied, a steroid systemically applied, a monoclonal antibody or antagonist to, e.g., interleukin 4 (Patent Literature 1: Japanese Patent No. 6463351), interferon (Patent Literature 2: JP H09-104636 A), aspirin powder (Patent Literature 3: JP H10-203988 A) and/or hyaluronic acid (Non Patent Literature 3: Indian Journal of Otolaryngology and Head & Neck Surgery 67 (3): 299-307, September 2015).

In the meantime, a heparin having an anticoagulant effect has been reported to significantly suppress production of mucus and neutrophil infiltration in rat nasal mucosal inflammation models (Non Patent Literature 4: Otolaryngology Immune Allergy 29 (3): 221-227, 2011); however, a heparin did not show a reduction effect on nasal polyps surgically obtained from patients with eosinophilic sinusitis (Non Patent Literature 5: Allergology International 66 (2017) 594-602).

Citation List

Patent Literatures

    Patent Literature 1: Japanese Patent No. 6463351
    Patent Literature 2: JP H09-104636 A
    Patent Literature 3: JP H10-203988 A

Non Patent Literatures

    Non Patent Literature 1: Kawasaki Medical School Journal 35 (1): 39-50, 2009
    Non Patent Literature 2: Bulletin of the Japan Otolaryngology Society 2018, 121, 1118-1120

Non Patent Literature 3: Indian Journal of Otolaryngology and Head & Neck Surgery 67 (3): 299-307, September 2015
Non Patent Literature 4: Otolaryngology Immune Allergy 29 (3): 221-227, 2011
Non Patent Literature 5: Allergology International 66 (2017) 594-602

Summary of Invention

Technical Problem

The present invention provides a nasal polyps reducing agent which shows an excellent reduction effect on nasal polyps and is safe, or a method for treating nasal polyps.

Solution to Problem

As a result of intensive studies, the present inventors found that a predetermined polysaccharide, particularly a sulfated polysaccharide such as a heparinoid and pentosan polysulfate (PPS), significantly reduces nasal polyps. Based on the finding, the present invention was accomplished.
More specifically, the present invention is as described below.

(1) A nasal polyps reducing agent comprising a polysaccharide selected from a polysulfated chondroitin sulfate, chondroitin sulfate, dermatan sulfate, keratan sulfate, heparan sulfate, dextran sulfate, pentosan polysulfate, chondroitin, glucomannan, inulin and xylo-oligosaccharide, or a salt thereof, as an active ingredient.
(1a) The nasal polyps reducing agent according to (1), wherein the polysaccharide or a salt thereof is selected from a polysulfated chondroitin sulfate, chondroitin sulfate, keratan sulfate, heparan sulfate, dextran sulfate, pentosan polysulfate, chondroitin, glucomannan and inulin.
(1b) The nasal polyps reducing agent comprising a sulfated polysaccharide or a salt thereof as an active ingredient.
(1c) The nasal polyps reducing agent according to (1b), wherein the sulfated polysaccharide or a salt thereof is selected from a polysulfated chondroitin sulfate, chondroitin sulfate, keratan sulfate, heparan sulfate, dextran sulfate and pentosan polysulfate.
(2) The nasal polyps reducing agent according to (1b), wherein the sulfated polysaccharide is composed of units of a monosaccharide selected from D-galactosamine, D-glucuronic acid, L-iduronic acid, D-glucose, D-galactose, D-xylose and L-arabinose, which may be partially acetylated.
(3) The nasal polyps reducing agent according to (1b) or (2), wherein the sulfated polysaccharide is a polysulfated chondroitin sulfate, a polysulfated dermatan sulfate or pentosan polysulfate.
(4) The nasal polyps reducing agent according to any one of (1) to (3), the agent being administered intranasally.
(5) A pharmaceutical composition comprising a polysaccharide selected from a polysulfated chondroitin sulfate, chondroitin sulfate, dermatan sulfate, keratan sulfate, heparan sulfate, dextran sulfate, pentosan polysulfate, chondroitin, glucomannan, inulin and xylo-oligosaccharide, or a salt thereof, for use in reducing nasal polyps in a patient with chronic sinusitis.
(5a) The pharmaceutical composition according to (5), wherein the polysaccharide is selected from a polysulfated chondroitin sulfate, chondroitin sulfate, keratan sulfate, heparan sulfate, dextran sulfate, pentosan polysulfate, chondroitin, glucomannan and inulin.
(5b) A pharmaceutical composition comprising a sulfated polysaccharide or a salt thereof for use in reducing nasal polyps in a patient with chronic sinusitis.
(6) The pharmaceutical composition according to (5b), wherein the sulfated polysaccharide is composed of units of a monosaccharide selected from D-galactosamine, D-glucuronic acid, L-iduronic acid, D-glucose, D-galactose, D-xylose and L-arabinose, which may be partially acetylated.
(7) The pharmaceutical composition according to (5b) or (6), wherein the sulfated polysaccharide is a polysulfated chondroitin sulfate, a polysulfated dermatan sulfate or pentosan polysulfate.
(8) The pharmaceutical composition according to any one of (5) to (7), wherein the patient with chronic sinusitis is a patient with eosinophilic sinusitis or non-eosinophilic sinusitis.
(9) The pharmaceutical composition according to any one of (5) to (8), wherein the patient with chronic sinusitis is a patient with eosinophilic sinusitis.
(10) An intranasal formulation comprising the pharmaceutical composition according to any one of (5) to (9).
(11) A method for treating nasal polyps, comprising administering an effective amount of a polysaccharide selected from a polysulfated chondroitin sulfate, chondroitin sulfate, dermatan sulfate, keratan sulfate, heparan sulfate, dextran sulfate, pentosan polysulfate, chondroitin, glucomannan, inulin and xylo-oligosaccharide or a salt thereof, to a patient in need of treatment of nasal polyps.
(12) The method according to (11), wherein the polysaccharide or a salt thereof is selected from a polysulfated chondroitin sulfate, chondroitin sulfate, keratan sulfate, heparan sulfate, dextran sulfate, pentosan polysulfate, chondroitin, glucomannan and inulin.

(13) The method for reducing nasal polyps, comprising administering an effective amount of a sulfated polysaccharide or a salt thereof to a patient in need of treatment of nasal polyps.

(14) The method according to (13), wherein the sulfated polysaccharide or a salt thereof is selected from a polysulfated chondroitin sulfate, chondroitin sulfate, keratan sulfate, heparan sulfate, dextran sulfate and pentosan polysulfate.

(15) The method according to (13), wherein the sulfated polysaccharide is composed of units of a monosaccharide selected from D-galactosamine, D-glucuronic acid, L-iduronic acid, D-glucose, D-galactose, D-xylose and L-arabinose, which may be partially acetylated.

(16) The method according to (13), wherein the sulfated polysaccharide is a polysulfated chondroitin sulfate, a polysulfated dermatan sulfate or pentosan polysulfate.

(17) The method according to (11), wherein the patient has sinusitis.

(18) The method according to (11), wherein the patient has a chronic sinusitis such as eosinophilic sinusitis or non-eosinophilic sinusitis.

(19) The method according to (11), wherein the patient has eosinophilic sinusitis.

(20) The method according to (11), comprising intranasally administering.

Advantageous Effects of Invention

A predetermined polysaccharide, particularly a sulfated polysaccharide such as heparinoid and pentosan polysulfate, is useful as a safe nasal polyps reducing agent at a low dose.

Brief Description of Drawings

[Figure 1] The figure shows a nasal polyps weight change at different doses (0.3, 30 and 3,000 $\mu$g/mL) of a heparinoid.
[Figure 2] The figure shows a nasal polyps weight change at different doses (0.003, 0.03, 0.3 and 30 $\mu$g/mL) of a heparinoid.
[Figure 3] The figure shows a nasal polyps weight change at a dose (0.003 $\mu$g/mL) of pentosan polysulfate (PPS).

Description of Embodiments

The present invention will be more specifically described below.

The polysaccharide to be used in the present invention is a sulfated mucopolysaccharide selected from polysulfated mucopolysaccharides such as chondroitin sulfate, chondroitin, dermatan sulfate, keratan sulfate, heparan sulfate and a polysulfated chondroitin sulfate; a sulfated polysaccharide including dextran sulfate and pentosan polysulfate in addition to the sulfated mucopolysaccharide; and glucomannan, inulin and xylo-oligosaccharide. A polysaccharide is preferably selected from a polysulfated chondroitin sulfate, chondroitin sulfate, keratan sulfate, heparan sulfate, dextran sulfate, pentosan polysulfate, chondroitin, glucomannan and inulin.

The sulfated polysaccharide in the present invention is composed of units of a monosaccharide selected from D-glucosamine, D-galactosamine, D-glucuronic acid, L-iduronic acid, D-galacturonic acid, D-glucose, D-galactose, D-xylose and L-arabinose, which may be partially acetylated; in other words, a polysaccharide composed of one or more types of monosaccharides as a repeat unit and having a sulfate group(s). In the sulfated polysaccharide to be used in the present invention, a sulfate group is contained in an average ratio of 0.55 to 4, preferably 0.6 to 2.9, and more preferably 0.7 to 2, per molecule of a monosaccharide.

In the sulfated polysaccharide to be used in the present invention the content of a sulfate group is preferably 10 to 70 w/w% as an amount of organic sulfate group, which is calculated in accordance with the quantitation method defined in the column of "heparinoid" of Japanese Pharmaceutical Codex 2002. And the sulfated polysaccharide having 10 to 65 w/w% as an amount of organic sulfate group is more preferred.

The sulfated polysaccharide is preferably composed mainly of units of a monosaccharide selected from D-galactosamine, D-glucuronic acid, L-iduronic acid, D-glucose, D-galactose, D-xylose and L-arabinose, which may be partially acetylated; and more preferably composed of units of a monosaccharide selected from D-galactosamine, D-glucuronic acid, D-glucose, D-galactose, D-xylose and L-arabinose.

Note that, examples of the monosaccharide partially acetylated include a naturally occurring monosaccharide such as acetyl glucose, N-acetyl glucosamine, acetyl galactose, N-acetyl galactosamine and acetyl xylose.

The weight average molecular weight of the sulfated polysaccharide or a salt thereof to be used in the present invention is 1,000 to 10,000,000 and preferably 4,000 to 1,000,000.

The weight average molecular weight used herein is a value obtained by the following equation:

$$\text{Weight average molecular weight (Mw)} = \Sigma(N_i \cdot M_i^2)/\Sigma(N_i \cdot M_i)$$

where $M_i$ represents the molecular weight of a polymer and $N_i$ represents the number of polymers.

The weight average molecular weight refers to a value measured by gel permeation chromatography (GPC). The weight average molecular weight is a value converted based on the value of a standard substance such as pullulan or polyethylene glycol measured by GPC. The weight average molecular weight can be measured, for example, by high performance liquid chromatography (Waters or Shimadzu Corporation) using a column, Ohpak SB-804 and SB-803 (both are manufactured by Showa Denko K.K.). As the mobile phase, an aqueous medium, such as an ammonium acetate aqueous solution and a sodium chloride aqueous solution, can be used at a flow rate of 1.0 mL/min. Detection can be made by a method based on a differential refractive index.

The sulfated polysaccharides to be used in the present invention can be obtained by the following methods.

The sulfated polysaccharides can be each obtained by subjecting a polysaccharide composed of monosaccharide units which may be partially acetylated, to a sulfation reaction to introduce a sulfate group.

The sulfation reaction is carried out by preparing an ice-cooled solvent in an amount of 10 to 30 mL relative to 1 g of a raw-material polysaccharide, adding a sulfation agent to the solvent in an amount 2 to 6 times the raw material polysaccharide (1 g), adding 1 g of the raw-material polysaccharide to the solution, and allowing the mixture to react at 0°C to 100°C for 1 to 10 hours. Examples of the solvent that can be used herein include pyridine, N,N-dimethylformamide and N,N-dialkyl acrylamide. Examples of the sulfation agent that can be used herein include chlorosulfonic acid and a triethylamine-sulfur trioxide complex salt.

In the sulfated polysaccharide to be used in the present invention and composed of monosaccharide units which may be partially acetylated, the constituent sugar in molar ratio of 0% to 60% of the sulfated polysaccharide is preferably N-acetylated or O-acetylated.

In the present invention, a sulfated polysaccharide may be used in the form of a physiologically acceptable salt. Examples of the physiologically acceptable salt include an alkali metal salt such as a sodium salt and a potassium salt, an alkaline earth metal salt such as a calcium salt, and a magnesium salt. Use of these salts in plural may be also included.

Examples of the sulfated polysaccharide of the present invention include sulfated mucopolysaccharide, dextran sulfate and pentosan polysulfate. A polysulfated chondroitin sulfate, chondroitin sulfate, chondroitin, dermatan sulfate, keratan sulfate, heparan sulfate, dextran sulfate and pentosan polysulfate are preferable.

In the specification of the present application, a polysulfated mucopolysaccharide, such as chondroitin sulfate, chondroitin, dermatan sulfate, keratan sulfate, heparan sulfate and a polysulfated chondroitin sulfate, is classified as a sulfated mucopolysaccharide.

Glucomannan, inulin and xylo-oligosaccharide are classified as a polysaccharide not corresponding to those illustrated above.

The "mucopolysaccharide" refers to a long-chain amino sugar having a disaccharide repeat unit consisting of a hexosamine, and a uronic acid or galactose, as a basic structure.

The "sulfated mucopolysaccharide" refers to a mucopolysaccharide having a sulfate group. The sulfated mucopolysaccharide of the present invention includes a naturally occurring mucopolysaccharide having a sulfate group in the sugar chain; and a sulfated mucopolysaccharide obtained by further chemically modifying a mucopolysaccharide or a naturally occurring sulfated mucopolysaccharide.

The "polysulfated mucopolysaccharide" refers to a sulfated mucopolysaccharide having a larger number of sulfate groups in the sugar chain. Not only a naturally occurring polysulfated mucopolysaccharide but also a polysulfated mucopolysaccharide obtained by further chemically modifying a mucopolysaccharide or a naturally occurring sulfated mucopolysaccharide is included.

Examples of the sulfated mucopolysaccharide will be described below. The whole structures of the sulfated mucopolysaccharides vary depending on the types of proteoglycans formed of the sulfated mucopolysaccharides, the species, tissues and developmental stages of the animals where the sulfated mucopolysaccharides are present. More specifically, in some cases of naturally occurring mucopolysaccharides or sulfated mucopolysaccharides, a basic structure (described below) of a sulfated mucopolysaccharide is further modified, or a structure other than the basic structure and/or a sugar are partially present therein. The sulfated mucopolysaccharides to be used in the present invention include such variations.

The "hexosamine" refers to a wide variety of compounds obtained by substituting a hydroxy group of a hexose with an amino group, and more specifically, refers to, e.g., D-glucosamine and D-galactosamine.

The "uronic acid" refers to a wide variety of compounds obtained by oxidizing a primary alcohol (group) of an aldose to a carboxyl group, and more specifically, refers to a naturally occurring uronic acid such as D-glucuronic acid, L-iduronic acid and D-galacturonic acid.

"Chondroitin sulfate" refers to a substance isolated from animal's viscous secretions or cartilage tissues. Chondroitin sulfate A and chondroitin sulfate C, D and E are known, which vary in the binding position of a constituent sugar to a sulfate group. In the specification of the present application, chondroitin sulfate A and chondroitin sulfate C are classified as a sulfated mucopolysaccharide; whereas, chondroitin sulfate D and E are sulfated mucopolysaccharides, which are also classified as a polysulfated mucopolysaccharide.

Chondroitin sulfate A (chondroitin 4-sulfate) has a repeat unit consisting of a disaccharide of N-acetyl-D-galactosamine (GalNAc) having a sulfate group at the 4-position and D-glucuronic acid (GlcA), as a basic structure.

Chondroitin sulfate C (chondroitin 6-sulfate) has a repeat unit consisting of a disaccharide of GalNAc having a sulfate group at the 6-position and GlcA, as a basic structure.

Chondroitin sulfate A and C are preferable.

A low-molecular chondroitin sulfate having a weight average molecular weight as low as 100,000 or less, and preferably 10,000 to 50,000, can be used. A low-molecular chondroitin sulfate can be obtained by decomposing a naturally occurring chondroitin sulfate with an enzyme such as chondroitinase or chondroitin sulfate lyase.

"Dermatan sulfate" has a repeat unit consisting of a disaccharide iduronic acid (IdoUA) and GalNAc having a sulfate group at the 4-position, as a basic structure and is sometimes referred to as chondroitin sulfate B.

"Keratan sulfate" has a repeat unit consisting of a disaccharide of galactose (Gal) and N-acetyl-D-glucosamine (GlcNAc), which are alternately linked via $\beta(1\rightarrow4)$ and $\beta(1\rightarrow3)$ linkage, respectively, as a basic structure. Note that the 6-position of GlcNAc herein is always sulfated.

"Heparan sulfate" refers to a polysaccharide having a constituent sugar such as D-glucosamine, D-glucuronic acid or L-iduronic acid and substituted with N-acetyl, N-sulfate or O-sulfate group.

"Chondroitin" refers to a mucopolysaccharide having a repeat unit consisting of a disaccharide of GalNAc and GlcA, as a basic structure, and particularly low in number of sulfate groups (usually, 0.7 molecules or less per disaccharide unit). Chondroitin can be obtained not only from a naturally occurring substance such as bovine cornea but also by chemical desulfation of chondroitin sulfate.

As the sulfated mucopolysaccharide to be used in the present invention, a sulfated mucopolysaccharide having a repeat unit consisting of a disaccharide composed of N-acetyl-D-galactosamine or N-acetyl-D-glucosamine and uronic acid or galactose, is preferable, and chondroitin sulfate, dermatan sulfate, keratan sulfate and heparan sulfate are more preferable.

In the sulfated mucopolysaccharide to be used in the present invention, polysulfated mucopolysaccharides such as further sulfated chondroitin sulfate, dermatan sulfate and keratan sulfate, are included.

The number of sulfate groups in the sulfated mucopolysaccharide of the present invention, although it is not particularly limited, is usually 0.55 to 4 molecules, preferably 0.6 to 2.9 molecules, more preferably 0.7 to 2 molecules in average per monosaccharide.

The molecular weight of the sulfated mucopolysaccharide or a salt thereof to be used in the present invention is not limited since it varies depending on the type of polysaccharide. The average molecular weight thereof (in terms of weight average molecular weight) is 10,000 to 1,000,000, and preferably 10,000 to 50,000.

The source from which a sulfated mucopolysaccharide of the present invention is derived is not particularly limited. A more preferable sulfated mucopolysaccharide is, for example, obtained by artificial sulfation of a naturally occurring mucopolysaccharide (illustrated above).

The "polysulfated mucopolysaccharide" refers to a mucopolysaccharide having a larger number of sulfate groups substituted among the sulfated mucopolysaccharides (illustrated above) and more specifically, a polysulfated chondroitin sulfate and a polysulfated dermatan sulfate. A polysulfated chondroitin sulfate is preferable.

As a method for obtaining a polysulfated mucopolysaccharide by further introducing a sulfate group(s) to a sulfated mucopolysaccharide such as chondroitin sulfate A and C, a method known in the technical field is illustrated, such as a method of reacting a sulfated mucopolysaccharide in an appropriate solvent in the presence of a sulfation agent while heating. The sulfation agent is not particularly limited as long as it can attain desired polysulfation. A complex of anhydrous sulfuric acid and, e.g., pyridine or triethylamine, is preferably used. The ratio of the sulfation agent to be used, which can be arbitrarily selected in accordance with a desired sulfation rate (or sulfur content) of a sulfated mucopolysaccharide and reaction conditions, is generally 2 to 10 parts by weight relative to 1 part by weight of the sulfated mucopolysaccharide to be a polysulfated. Examples of the solvent include a protophilic solvent such as dimethylformamide. The temperature and time for the reaction, which are not particularly limited as long as a desired sulfation rate can be attained, are, for example, 40 to 90°C and 30 minutes to 20 days.

A polysulfated mucopolysaccharide produced as described above can be purified by a purification operation routinely used for modified polysaccharides. Examples of the purification operation include neutralization, desalting by dialysis, collecting precipitations by addition of an organic solvent, and collection by lyophilization.

In the present invention, a polysulfated mucopolysaccharide may be used in the form of a physiologically acceptable salt. Examples of the physiologically acceptable salt include an alkali metal salt such as a sodium salt and a potassium salt, an alkaline earth metal salt such as calcium salt, and a magnesium salt. Use of these salts in plural may be also included.

The "polysulfated chondroitin sulfate" refers to a polymer having 2 to 4 and preferably 2 to 3 sulfate groups per disaccharide unit consisting of D-acetylgalactosamine and D-glucuronic acid.

Examples of the polysulfated chondroitin sulfate include chondroitin sulfate D, chondroitin sulfate E and a heparinoid listed in Japanese Pharmaceutical Codex. A heparinoid listed in Japanese Pharmaceutical Codex is preferable.

As the polysulfated chondroitin sulfate, a naturally occurring polysulfated chondroitin sulfate such as chondroitin sulfate D or chondroitin sulfate E may be used. Other than the naturally occurring one, a polysulfated chondroitin sulfate can be easily produced in accordance with a method (known in the technical field) by allowing a chondroitin component such as chondroitin or chondroitin sulfate (A, C, D, E) to react with a sulfation agent such as a chlorosulfuric acid, concentrated sulfuric acid and a sulfur trioxide-pyridine complex.

As a preferable polysulfated chondroitin sulfate, a heparinoid listed in Japanese Pharmaceutical Codex can be illustrated.

More specifically, a polysulfated chondroitin sulfate having the physicochemical properties represented by the following values is used.

    a) Content of sulfate group: 25.8 to 37.3%
    b) Limiting viscosity: 0.09 to 0.18

A polysulfated chondroitin sulfate may be used in the form of a free acid derived from a sulfate residue and usually a base salt thereof is used.

Examples of the base salt include an alkali metal salt such as a sodium salt and a potassium salt, an alkaline earth metal salt such as calcium salt, and a magnesium salt.

Chondroitin sulfate D is isolated from, e.g., shark cartilage, and has a similar structure to that of chondroitin sulfate C. As a basic structure thereof, a sulfate group is present at not only the 6-position of GalNAc but also the 2- or 3-position of GlcA.

Chondroitin sulfate E (chondroitin 4-, 6-sulfate) is isolated from, e.g., surumeika squid cartilage, and has a similar structure to that of chondroitin sulfate C. As a basic structure thereof, a sulfate group is present at the 4- and 6-positions of GalNAc.

The "polysulfated dermatan sulfate" refers to a synthesized polysulfated dermatan sulfate obtained by chemically introducing a sulfate group into dermatan sulfate, which is a naturally occurring sulfated mucopolysaccharide having a repeat unit consisting of a sugar composed of N-acetylgalactosamine and L-iduronic acid; a naturally occurring dermatan polysulfate; and a synthesized polysulfated dermatan sulfate obtained by chemically introducing a sulfate group into a naturally occurring dermatan polysulfate.

The number of sulfate groups to be introduced, although it is not particularly limited, is for example, more than one to up to 4, preferably 1.3 to 4, and more preferably 2 to 4, per sugar repeat unit. The weight average molecular weight of a polysulfated dermatan sulfate of the present invention is, for example, 1,000 to several tens of thousands.

The other sulfated polysaccharides to be used in the present invention will be described below.

"Dextran sulfate" is a polyanion derivative of dextran, which is a polysaccharide composed of glucose units each constituting of a linear-chain of glucoses linked via $\alpha$ (1→6) linkage and a branch chain starting from $\alpha$ (1→3) linkage different in length (usually 1,000 to several thousands to hundreds of thousands of Daltons (Da)), and which is sulfated at the C2 to C4 positions and partially at the C1 and C6 positions of a terminal group.

"Pentosan polysulfate" is a semi-synthesized sulfated polysaccharide containing a mixture of polyvalent anionic polysaccharides having D-xylose and/or L-arabinose as a constituent monosaccharide(s). Pentosan polysulfate is produced by chemical sulfation of a polysaccharide (for example, xylan) obtained from an arbor, for example, beech, and generally considered as a polysulfate obtained by linking O-methyl glucuronic acid to a xylan chain as a side chain.

Pentosan polysulfate may be used in the form of a physiologically acceptable salt. Examples of the physiologically acceptable salt include an alkali metal salt such as a sodium salt and a potassium salt, an alkaline earth metal salt such as calcium salt, and a magnesium salt. A particularly preferable salt is sodium pentosan polysulfate.

Although pentosan polysulfate is not particularly limited, pentosan polysulfate has a weight average molecular weight of preferably 1,000 to 30,000, more preferably 2,000 to 10,000 and further preferably 4,000 to 6,500.

Now, polysaccharides other than sulfated polysaccharides will be more specifically described, below.

"Glucomannan" is primarily composed of glucose and mannose, which link via $\beta$(1→4) linkage to form a main chain, and partially has a branched structure via $\beta$(1→3) linkage and $\beta$(1→6) linkage.

"Inulin" is a substance obtained by linking 2 to 140 molecules of fructose via $\beta$(2→1) linkage and having glucose at the end.

The "xylo-oligosaccharide", which is a hydrolyzate of xylan, is an oligosaccharide having a structure formed of 2 to 7 xylose molecules linked via β(1→4) linkage. Xylo-oligosaccharides having a 4-O-methyl glucuronic acid and an arabinofuranosyl derived from a raw-material, xylan, as a side chain, are also included.

The polysaccharides illustrated above may be used in the form of a physiologically acceptable salt. Examples of the physiologically acceptable salt include an alkali metal salt such as a sodium salt and a potassium salt, an alkaline earth metal salt such as calcium salt, and a magnesium salt. Use of these salts in plural may be also included.

The polysaccharides illustrated above can be extracted/collected from animal and plant tissues, microorganisms such as *Streptococcus* microbe, cultures of animal cells or plant cells. Also, commercially available polysaccharides can be used.

According to the present invention, it is possible to provide a medical drug containing a predetermined polysaccharide as illustrated above, preferably a predetermined sulfated polysaccharide, as an active ingredient, and effectively reducing nasal polyps.

The medical drug of the present invention, since it has a reduction effect on nasal polyps, can be expected to improve symptoms of a diseases with nasal polyps, more specifically, chronic sinusitis including eosinophilic sinusitis and non-eosinophilic sinusitis, and symptoms of allergic rhinitis.

According to another embodiment of the present invention, there is provided a method for treating nasal polyps, a method for reducing nasal polyps or a method for preventing or treating nasal polyps by administering a predetermined polysaccharide of the present invention to a patient in need of treatment of nasal polyps.

Nasal polyps, which is also called as rhinopolypus, is a fleshy outgrowth of the mucous membrane that forms at the site (usually around the ostia of the maxillary sinuses) of dependent edema in the lamina propria. The patient in need of treatment of nasal polyps are a patient affected with a disease having nasal polyps, more specifically, a patient with sinusitis, preferably chronic sinusitis. Examples of chronic sinusitis include eosinophilic sinusitis and non-eosinophilic sinusitis.

Treating nasal polyps includes improving a symptom such as nasal congestion caused by nasal polyps, reducing or treating nasal polyps, in other words, reducing nasal polyps in size and improving a symptom such as nasal congestion.

Improvement of a symptom such as nasal congestion caused by nasal polyps refers to improvement of a clinical symptom such as nasal congestion, compared to a non-administration group of a polysaccharide in the present invention even if the number and size of nasal polyps are not reduced.

Reduction of nasal polyps means that at least one of number and size (area or weight of a single nasal polyp) of nasal polyps reduces.

The concentration of the polysaccharide to be used in the present invention in a liquid is preferably $3 \times 10^{-7}$ to 30 w/v% and more preferably 0.003 to 10 w/v%.

The polysaccharide to be used in the present invention is intranasally administered and delivered in the nasal cavity having nasal polyps, in other words, used as an intranasal formulation.

The intranasal formulation refers to a dosage form for intranasal administration; more specifically, a liquid, a suspension, a powder, a solid agent or a semi-solid agent. The suspension is a liquid formulation having solid particles dispersed in a liquid medium.

A preferable dosage form of the intranasal formulation is a nasal drop.

The nasal drop is a formulation which is administered to the nasal cavity or the nasal mucosa. Nasal drops are classified as a nasal liquid and a nasal powder. A nasal drop can be sprayed/inhaled, if necessary, by use of a device such as a spray pump being capable of spraying with an appropriate amount of drug equally. The dose of a nasal drop can be controlled by adjusting the amount to be released by the spray device.

The nasal liquid is a liquid formulation to be administered to the nasal cavity. Alternatively, the nasal liquid may be a liquid form, or solid form which is used by dissolving or suspending it when used. The nasal liquid is usually prepared by adding an active ingredient as it is or adding it together with a solvent or an appropriate additive, dissolving or suspending the resultant mixture, and if necessary, filtering the resultant solution. Alternatively, the nasal formulation is dissolved or suspended with an appropriate solvent or suspending liquid when used. To a nasal liquid, if necessary, an additive such as a dissolution aid, a tonicity agent, a buffer or a pH regulator can be added. In the case of a suspension, an additive such as a dispersant or a stabilizer can be added if necessary, in order to homogenize an active ingredient.

Examples of a nasal liquid dosage-form (means) include a nasal drop spray, a nose aerosol and a nose nebulizer. The nasal drop spray contains a polysaccharide usually dissolved or suspended in a solution or a mixture in a non-pressurized dispenser. A nasal spray is advantageous since the spray device is small and convenience, and used in a simple manner, and a dose of 25 to 200 μL can be accurately measured and delivered. As a nasal spray, a liquid or suspension containing a polysaccharide can be used. Another dosage form for intranasal administration is a nose aerosol. In the nose aerosol spray, a drug is dispensed by excessive pressure and released through a valve. In a nasal spray, a drug is forcibly dispensed by means of a micro pump bucket but the pressure of a vial is the same as atmospheric pressure. The nose aerosol has the same advantages as in the nasal spray.

The nose nebulizer is an administration means for administrating a drug in the state of fine mist ultrasonically produced.

When a multiple dose container is charged with a nasal liquid, if necessary, an appropriate preservative is added in an amount sufficient to inhibit growth of microorganisms. The container for a nasal liquid is usually an airtight container.

The nasal powder is a nasal drop in the form of fine powder that is administered to the nasal cavity and usually prepared by appropriately pulverizing an active ingredient into fine particles, mixing, if necessary, with an appropriate additive(s) and homogenizing them. A container for a nasal powder is usually an airtight container, if necessary, having moisture proof characteristic.

Other than nasal drops, a medical drug of the present invention may have a solid or a semi-solid dosage form such as an ointment, a plaster, a cream and a gel, or may be a highly viscous liquid or suspension suitable for application onto the nasal mucosa. These may be administered by applying them on the mucosa in the nasal cavity.

These dosage forms such as a nasal drop, an ointment, a plaster, a cream and a gel are prepared from the polysaccharide to be used in the present invention together with pharmaceutically acceptable additive(s).

As the pharmaceutically acceptable additives to be used in a medical drug of the present invention, additives usually used in the technical field, more specifically, pharmaceutical excipients and pharmaceutical carriers described below can be illustrated. A pharmaceutical composition appropriate for each dosage form can be prepared by appropriately using these additives in accordance with a method routinely used.

The pharmaceutically acceptable additives to be used in a medical drug of the present invention are not particularly limited as long as they are usually used in the technical field. Examples of the pharmaceutically acceptable additives include a base, a dissolution aid, an excipient, a dispersant, an emulsifier, a viscous agent, a tonicity agent, a buffer, a pH regulator, a stabilizer, a chelating agent, a preservative, an antioxidant, an absorption promoter, a moisturizing agent, a filler, a crosslinking agent, a cooling agent and a coating agent. A pharmaceutical composition appropriate for each dosage form can be prepared by appropriately using these additives in accordance with a method routinely used.

Examples of the base to be used in a medical drug of the present invention include a hydrophobic base, a hydrophilic base and water.

Examples of the hydrophobic base include, but are not particularly limited to, a higher hydrocarbon, a fat and oil, a wax, a fatty acid, a higher alcohol and a fatty acid ester. Examples of the higher hydrocarbon include squalane, synthetic paraffin, liquid paraffin, white petrolatum and microcrystalline wax. Examples of the fat and oil include sesame oil, corn oil, olive oil and cocoa butter. Examples of the wax include beeswax, white beeswax, lanolin, hydrogenated lanolin and ceresin wax. Examples of the fatty acid include stearic acid and oleic acid. Examples of the higher alcohol include lanolin alcohol, myristyl alcohol, cetyl alcohol, stearyl alcohol, cetostearyl alcohol and cholesterol. Examples of the fatty acid ester include isopropyl myristate, stearyl myristate and medium chain fatty acid triglyceride.

Examples of the hydrophilic base include ethanol, propanol, isopropanol, butanol, iso-butanol, propylene glycol, polyethylene glycol and macrogol.

Examples of the dissolution aid include, but are not limited to, propylene glycol, D-mannitol, benzyl benzoate, ethanol, isopropanol, triethanolamine, sodium carbonate and sodium citrate.

In the case where the medical drug of the present invention is a nasal powder, an excipient is desirably used as an additive.

Examples of the excipient include, but are not particularly limited to, a sugar alcohol such as erythritol, maltitol, mannitol, sorbitol, xylitol and lactitol; a sugar such as white sugar, lactose, hydrogenated maltose starch syrup, powder hydrogenated maltose starch syrup, glucose and maltose; cornstarch, crystalline cellulose, calcium monohydrogen phosphate, calcium hydrogen phosphate, anhydrous calcium hydrogen phosphate, light anhydrous silicic acid, hydrous silicon dioxide and silicon dioxide.

In the case where the medical drug of the present invention is a suspension, a dispersant is desirably used as an additive.

Examples of the dispersant include, but are not particularly limited to, a cellulose such as methyl cellulose, sodium carboxymethyl cellulose and hydroxypropyl methyl cellulose; a synthetic polymer compound such as polyvinyl alcohol, polyvinylpyrrolidone and carboxyvinyl polymer; a nonionic surfactant such as a polyoxyethylene alkyl ether, a polyoxyethylene polypropylene alkyl ether, a sorbitan fatty acid ester, a polyoxyethylene sorbitan fatty acid ester, a glycerin fatty acid ester, a polyglycerin fatty acid ester, a polyoxyethylene glycerin fatty acid ester, a polyethylene glycol fatty acid ester, a sucrose fatty acid ester, a polyoxyethylene castor oil, a polyoxyethylene hydrogenated castor oil and a polyoxyethylene polyoxypropylene polymer; an amphoteric surfactant such as an alkyl betaine, an alkylamide betaine, an alkyl sulfobetaine and imidazoline; and an anionic surfactant such as a saturated higher fatty acid salt, an alkyl sulfonate, an alkyl ether sulfonate and a polyoxyethylene alkyl ether phosphate.

Examples of the emulsifier include a cationic surfactant, an anionic surfactant, an amphoteric surfactant and a nonionic surfactant.

Examples of the cationic surfactant include, but are not particularly limited to, cetyltrimethylammonium chloride, lauryldimethylbenzylammonium chloride, tetrabutylammonium chloride and dioctadecyldimethylammonium chloride.

Examples of the anionic surfactant include, but are not particularly limited to, a sodium alkylbenzene sulfonate, sodium dodecyl sulfate, sodium palm-alcohol ethoxy sulfate, sodium $\alpha$-olefin sulfonate and emulsified cetostearyl alcohol.

Examples of the nonionic surfactant include, but are not particularly limited to, a polyoxyethylene alkyl ether, a polyoxyethylene alkylphenol ether, a polyoxyethylene sorbitan fatty acid ester, a polyoxyethylene hydrogenated castor oil, a polyoxyl stearate, a glycerin fatty acid ester and a diglycerin fatty acid ester.

Examples of the amphoteric surfactant include, but are not particularly limited to, N-alkyl-N,N-dimethylammonium betaine and an imidazoline amphoteric surfactant.

The surfactants illustrated above can be used alone or in combination.

Examples of the viscous agent include, but are not particularly limited to, sodium alginate, gelatin, methyl cellulose, a carboxyvinyl polymer, carboxymethyl cellulose and a sodium polyacrylate.

Examples of the tonicity agent include, but are not particularly limited to, a sugar such as sorbitol, glucose and mannitol; a polyvalent alcohol such as glycerin, polyethylene glycol and propylene glycol; and an inorganic salt such as sodium chloride and potassium chloride.

Examples of the buffer include, but are not particularly limited to, boric acid, borax, sodium monohydrogen phosphate, sodium dihydrogen phosphate, citric acid, sodium citrate, sodium dihydrogen citrate and disodium citrate.

Examples of the pH regulator include, but are not particularly limited to, diisopropanolamine, triisopropanolamine, triethanolamine, potassium hydroxide, sodium hydroxide, sodium citrate, phosphoric acid, tartaric acid, dl-malic acid and glacial acetic acid.

Examples of the stabilizer include, but are not particularly limited to, sodium edetate, sodium oleate, casein and sodium caseinate.

Examples of the chelating agent include, but are not particularly limited to, edetic acid, oxalic acid, citric acid, pyrophosphoric acid, hexametaphosphate, gluconic acid and a salt thereof.

Examples of the preservative include, but are not particularly limited to, a quaternary ammonium salt such as benzalkonium chloride, benzoxonium chloride, benzododecinium bromide, benzethonium chloride, cetylpyridinium chloride and cetrimide; a C1-C7 alkyl ester of 4-hydroxybenzoic acid and a salt thereof such as benzoic acid and a salt thereof, methyl 4-hydroxybenzoate and propyl 4-hydroxybenzoate; chlorhexidine and an intranasally acceptable salt thereof such as chlorhexidine digluconate, chlorhexidine acetate and chlorhexidine chloride; 2-phenylethanol, 2-phenoxyethanol, chloro-butanol and sorbic acid.

As the preservative, an intranasally acceptable preservative is preferable.

Examples of the antioxidant include, but are not particularly limited to, polyphenol, ascorbic acid, t-butylhydroquinone, butylated hydroxyanisole, butylated hydroxytoluene, L-cysteine hydrochloride, sodium hydrogen sulfite, and $\alpha$-tocopherol and a derivative thereof.

Examples of the absorption promoter include, but are not particularly limited to, diisopropyl adipate, lecithin, squalane, squalene, I-menthol, polyethylene glycol, isopropyl myristate, dimethyl sulfoxide, mint oil, eucalyptus oil, d-limonene and dl-limonene.

Examples of the moisturizing agent include, but are not particularly limited to, glycerin, propylene glycol and 1,3-butylene glycol.

Examples of the filler include, but are not particularly limited to, kaolin, titanium dioxide and zinc oxide.

Examples of the crosslinking agent include, but are not particularly limited to, acetaldehyde, dimethyl ketone and aluminum sulfate.

Examples of the cooling agent include, but are not particularly limited to, I-menthol, dl-camphor, d-borneol, fennel oil, mint oil and mint water.

The nasal liquid containing the medical drug of the present invention may contain an intranasally acceptable film-forming agent. The film-forming agent, if added, suppresses water loss and maintains the hydration level of the nasal mucosa satisfactorily for a long time, with the result that the moisturizing effect and soothing effect of the composition of the present invention can be enhanced.

Examples of the coating agent include, but are not particularly limited to, a water soluble or water-swelling cellulose material such as hydroxypropyl methyl cellulose, hydroxypropyl cellulose, methyl cellulose, hydroxyethyl methyl cellulose and sodium carboxymethyl cellulose; polyvinylpyrrolidone (povidone) and cross-linked polyvinylpyrrolidone (crospovidone).

The medical drug of the present invention is not particularly limited since it varies depending on the age, body weight and sex of the patient and the severity of nasal polyps (amount, range). The dose of the medical drug varies depending on the severity of nasal polyps.

The medical drug is applied to the nasal cavity in a dose of $5 \times 10^{-11}$ to 1 g per day, once to several times.

Note that the disease state of chronic sinusitis, particularly eosinophilic sinusitis, is closely tied to asthma. To a chronic sinusitis patient with asthma, the medical drug of the present invention is applied in conjunction with an asthma agent.

Examples of the asthma agent are as follows:

Inhaled corticosteroid or steroid for nasal spray: beclomethasone propionate, fluticasone propionate, budesonide, Ciclesonide and mometasone furan carboxylate;

Combination drug of inhaled corticosteroid and a long-acting β2 stimulant: a combination drug with salmeterol xinafoate and a combination drug with formoterol fumarate hydrate;

theophylline sustained-release formulation: Theodur, Theolong, Slobid, Uniphyl, Unicon, Neophyllin, and Theodrip; Short-acting theophylline drug: Neophyllin, Theocolin, Monophylline, asthmolysin-D/M, asthphyllin and Albina suppository;

Long-acting β2 stimulant (LABA): an inhaled drug such as Serevent, a patch such as Hokunalin tape, and an oral drug such as Meptin, Spiropent, Hokunalin, Berachin, Atock and Broncholin;

Short-acting β2 stimulant (SABA): an inhaled drug such as Airomir, Sultanol, Meptin air and Berotec Aerosol; and an oral drug such as Venetlin, Alotec, Inolin, LEANOL, ephedrine and Isopal P;

Leukotriene antagonist (receptor antagonist): Onon, Accolate, Singulair and Kipres;

Th2 cytokine inhibitor: IPD;

Histamine H1 receptor antagonist: Zesulan, Nipolazin, Zaditen, Celtect and Alesion;

Mediator release inhibitor: Intal, Rizaben, Solfa, Romet, Ketas, Alegysal, Pemilaston, Tazanol and Tazalest;

Thromboxane A2 inhibitor: Vega and DOMENAN;

Thromboxane A2 antagonist: Bronica and Baynas;

Oral corticosteroid: predonine, prednisolone, Medrol, rinderon, Ledercort, Decadron, Corson, dexamethasone and Paramesone;

Immunosuppressive drug: cyclosporine;

Interleukin 4R (IL-4R) inhibitor: Dupilumab;

Iodine formulation: Jolethin.

The medical drug of the present invention can be administered in combination with a vasoconstrictor. Examples of the vasoconstrictor include naphazoline hydrochloride, tetrahydrozoline hydrochloride, phenylephrine hydrochloride, epinephrine hydrochloride, dl-methylephedrine hydrochloride, tetrahydrozoline nitrate, naphazoline nitrate and epinephrine.

Examples

The present invention will be more specifically described based on Examples, below. Note that, the present invention is not limited to the following Examples.

[Example 1] The reduction effect of heparinoid on nasal polyps from patient with eosinophilic sinusitis

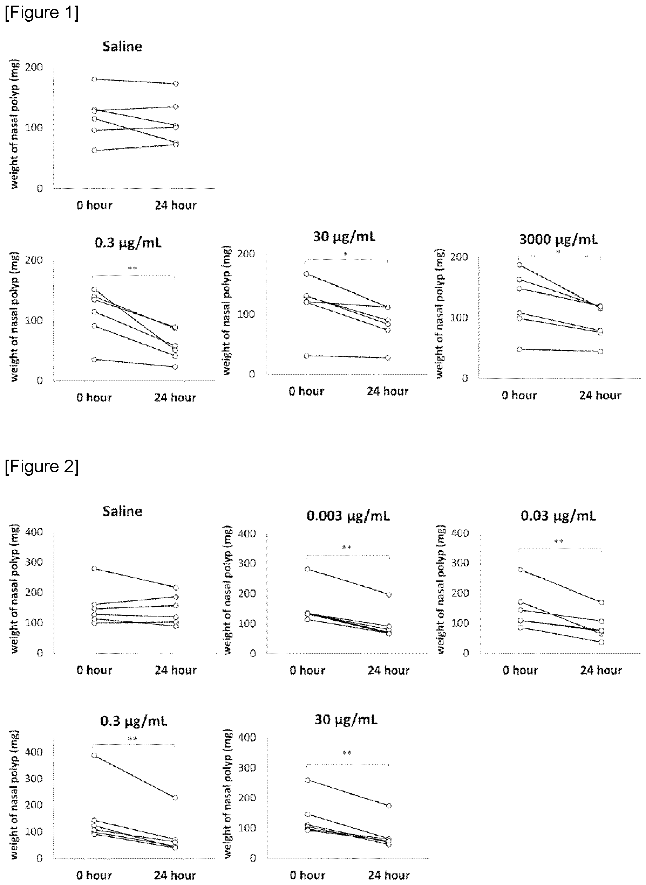

A heparinoid (organic sulfate group 25.8 to 37.3% w/w, D-glucuronic acid 19.0 to 24.0% w/w, Maruho Co., Ltd.) dried under reduced pressure was dissolved in saline to prepare heparinoid solutions (0.3, 30 and 3,000 μg/mL). A nasal polyps specimen was surgically obtained from a patient with eosinophilic sinusitis, cut into pieces of about 5 mm squares, wiped off the moisture and measured the weight. Each specimen was transferred to a 12-well plate. The specimen in the plate was incubated with 1 mL of saline or heparinoid solutions at 37°C for about 24 hours. Then, after wiping off the moisture in the incubated specimen taken from the plate, the weight of the specimen was measured. The difference between specimen weights before and after incubation was used as an index of a nasal polyps reduction effect. The saline group and heparinoid solution groups (different in concentration) of Figure 1 each consisted of 6 cases.

Changes in specimen weight before and after the incubation are shown in Figure 1. In Figure 1, symbols * and ** represent significant differences (*$P < 0.05$, ** $P < 0.01$) of nasal polyps weight before and after the incubation by a paired t-test. The weight of the nasal polyps incubated with saline was not changed significantly, whereas the weight of the nasal polyps incubated with heparinoid solutions (0.3, 30 and 3,000 μg/mL) was decreased significantly. From the results, it was suggested that the heparinoid has a reduction effect on nasal polyps.

[Example 2] The reduction effect of heparinoid on nasal polyps from patient with eosinophilic sinusitis

The reduction effects of heparinoid solutions (0.003, 0.03, 0.3 and 30 μg/mL) on nasal polyps were evaluated in the same method as in Example 1. The saline group and heparinoid solution groups (different in concentration) of Figure 2 each consisted of 6 cases.

Changes in specimen weight before and after the incubation are shown in Figure 2. In Figure 2, symbol ** represents significant difference (** $P < 0.01$) of nasal polyps weight before and after the incubation by a paired t-test. The weight of the nasal polyps incubated with saline was not changed significantly, whereas the weight of the nasal polyps incubated with heparinoid solutions (0.003, 0.03, 0.3 and 30 μg/mL) was decreased significantly. From the results, it was suggested that the heparinoid has a reduction effect on nasal polyps.

[Example 3] The reduction effect of pentosan polysulfate (PPS) on nasal polyps from patient with eosinophilic sinusitis

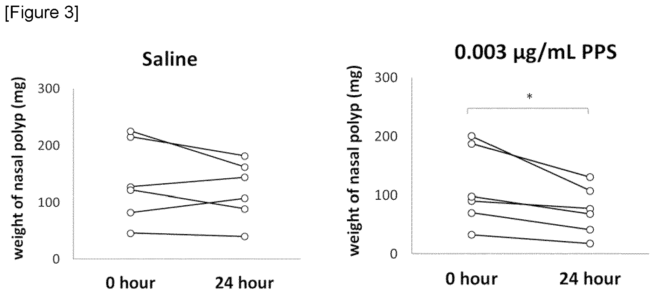

A PPS solution (0.003 μg/mL) was prepared and the reduction effect on nasal polyps was evaluated in the same method as in Example 1. PPS used herein was sodium pentosan polysulfate (weight average molecular weight 4,000 to 6,500, sulfur content 13.0 to 20.0%w/w, glucuronic acid content 2.5 to 4.0% w/w) manufactured by Molclone Labs. The saline group and PPS solution group of Figure 3 each consisted of 6 cases.
Changes in specimen weight before and after the incubation are shown in Figure 3. The weight of the nasal polyps incubated with saline was not changed significantly, whereas the weight of the nasal polyps incubated with 0.003 μg/mL PPS solution was decreased significantly. From the results, it was suggested that the PPS has a reduction effect on nasal polyps in patients with eosinophilic sinusitis.

[Example 4] The reduction effect of polysaccharides on nasal polyps from patient with eosinophilic sinusitis

The following 11 types of polysaccharide solutions (polysaccharides except glucomannan: 300 μg/mL; glucomannan 30 μg/mL) were prepared and the reduction effect on nasal polyps was evaluated in the same method as in Example 1. The saline groups and polysaccharide solution groups (different in concentration) of Tables 1 to 4 each consisted of a single case.

- Chondroitin (chondroitin sodium, weight average molecular weight 42,351, sulfate group content 2.6%, manufactured by Maruho Co., Ltd.)
- Low-molecular chondroitin sulfate (low-molecular sodium chondroitin sulfate, weight average molecular weight 11,500, sulfate group content 8.9%, manufactured by Maruho Co., Ltd.)
- Chondroitin sulfate A (sodium chondroitin sulfate A, manufactured by PG Research)
- Dermatan sulfate (sodium dermatan sulfate, manufactured by Tokyo Chemical Industry Co., Ltd.)
- Chondroitin sulfate C (sodium chondroitin sulfate C, manufactured by PG Research)
- Glucomannan (Propol A, manufactured by Shimizu Chemical Corporation)
- Dextran sulfate (sodium dextran sulfate 500,000, manufactured by FUJIFILM Wako Pure Chemical Corporation)
- Keratan sulfate (sodium keratan sulfate, manufactured by PG Research)
- Inulin (manufactured by Tokyo Chemical Industry Co., Ltd.)
- Heparan sulfate (manufactured by Toronto Research Chemicals, Inc.)
- Xylo-oligosaccharide (xylo-hexaose, manufactured by Megazyme)

Chondroitin was synthesized by desulfurization of sodium chondroitin sulfate (manufactured by Bioiberica) in the same method as in JP H07-062001 A. Low-molecular chondroitin sulfate was synthesized by hydrolysis of sodium chondroitin sulfate (manufactured by Bioiberica) in acidic conditions. The other polysaccharides used herein were commercially available products.
Changes in specimen weight before and after the incubation are shown in Tables 1 to 4. The weight of the nasal polyps incubated with saline was almost not changed, whereas the weight of the nasal polyps incubated each with 11 types of polysaccharide solutions (polysaccharides except glucomannan: 300 μg/mL; glucomannan 30 μg/mL) was remarkably decreased. From the results, it was suggested that the 11 types of polysaccharide solutions have a reduction effect on nasal polyps in patients with eosinophilic sinusitis.

[Table 1]

|  | Before incubation (mg) (A) | After incubation (mg) (B) | (A) - (B) (mg) |
|---|---|---|---|
| Saline | 132.0 | 126.0 | 6.0 |
| Chondroitin | 154.8 | 122.0 | 32.8 |
| Chondroitin sulfate A | 106.0 | 68.0 | 38.0 |
| Chondroitin sulfate C | 124.0 | 80.0 | 44.0 |

[Table 2]

|  | Before incubation (mg) (A) | After incubation (mg) (B) | (A) - (B) (mg) |
|---|---|---|---|
| Saline | 100.0 | 104.0 | -4.0 |
| Glucomannan | 100.0 | 81.0 | 19.0 |
| Dextran sulfate | 142.0 | 120.0 | 22.0 |

[Table 3]

|  | Before incubation (mg) (A) | After incubation (mg) (B) | (A) - (B) (mg) |
|---|---|---|---|
| Saline | 152.0 | 163.0 | -11.0 |
| Keratan sulfate | 165.6 | 147.3 | 18.3 |

[Table 4]

|  | Before incubation (mg) (A) | After incubation (mg) (B) | (A) - (B) (mg) |
|---|---|---|---|
| Saline | 83.0 | 75.0 | 8.0 |
| Dermatan sulfate | 131.6 | 118.5 | 13.1 |
| Low-molecular chondroitin sulfate | 141.0 | 116.0 | 25.0 |
| Inulin | 131.4 | 85.3 | 46.1 |
| Heparan sulfate | 128.5 | 103.0 | 25.5 |
| Xylo-oligosaccharide | 128.3 | 110.4 | 17.9 |

[Example 5] The reduction effect of heparinoid and PPS on nasal polyps from a patient with non-eosinophilic sinusitis

In the same method as in Example 1, heparinoid solutions and PPS solutions (0.03, 0.3, 30 and 300 $\mu$g/mL, respectively) were prepared and the reduction effect on nasal polyps specimen surgically obtained from a patient with non-eosinophilic sinusitis was evaluated. The heparinoid and PPS were also the same as those used in Example 1 and Example 3, respectively. The saline groups, heparinoid solution group and PPS solution groups of Table 5 each consisted of a single case.

Changes in specimen weight before and after incubation are shown in Table 5. The weight of the nasal polyps incubated with saline was not decreased, whereas the weight of the nasal polyps incubated with the heparinoid solution and the PPS solutions (0.03, 0.3, 30 and 300 $\mu$g/mL, individually) was remarkably decreased. From the results, it was suggested that the heparinoid and PPS also have a reduction effect on nasal polyps in patients with non-eosinophilic sinusitis.

[Table 5]

|  | Before incubation (mg) (A) | After incubation (mg) (B) | (A) - (B) (mg) |
|---|---|---|---|
| Saline | 300.0 | 322.0 | -22.0 |
| 0.03 $\mu$g/mL heparinoid | 311.0 | 276.0 | 35.0 |
| 0.3 $\mu$g/mL heparinoid | 209.0 | 181.0 | 28.0 |
| 30 $\mu$g/mL heparinoid | 235.0 | 169.0 | 66.0 |
| 300 $\mu$g/mL heparinoid | 276.0 | 246.0 | 30.0 |
| 0.03 $\mu$g/mL PPS | 233.0 | 214.0 | 19.0 |
| 0.3 $\mu$g/mL PPS | 273.0 | 225.0 | 48.0 |
| 30 $\mu$g/mL PPS | 220.0 | 207.0 | 13.0 |

(continued)

|  | Before incubation (mg) (A) | After incubation (mg) (B) | (A) - (B) (mg) |
|---|---|---|---|
| 300 μg/mL PPS | 125.0 | 115.0 | 10.0 |

Industrial Applicability

Since predetermined polysaccharides including a sulfated polysaccharide, i.e., heparinoid, exerted significant effects on nasal polyps at low dose, these are useful as a nasal polyps reducing agent for treating or preventing chronic sinusitis associated with nasal polyps.

**Claims**

1. A nasal polyps reducing agent comprising a polysaccharide selected from a polysulfated chondroitin sulfate, chondroitin sulfate, dermatan sulfate, keratan sulfate, heparan sulfate, dextran sulfate, pentosan polysulfate, chondroitin, glucomannan, inulin and xylo-oligosaccharide, or a salt thereof, as an active ingredient.

2. The nasal polyps reducing agent according to claim 1, wherein the polysaccharide is selected from a polysulfated chondroitin sulfate, chondroitin sulfate, keratan sulfate, heparan sulfate, dextran sulfate, pentosan polysulfate, chondroitin, glucomannan and inulin.

3. The nasal polyps reducing agent according to claim 1 or 2, the agent being administered intranasally.

4. A pharmaceutical composition comprising a polysaccharide selected from a polysulfated chondroitin sulfate, chondroitin sulfate, dermatan sulfate, keratan sulfate, heparan sulfate, dextran sulfate, pentosan polysulfate, chondroitin, glucomannan, inulin and xylo-oligosaccharide or a salt thereof, for use in reducing nasal polyps in a patient with chronic sinusitis.

5. The pharmaceutical composition according to claim 4, wherein the polysaccharide is selected from a polysulfated chondroitin sulfate, chondroitin sulfate, keratan sulfate, heparan sulfate, dextran sulfate, pentosan polysulfate, chondroitin, glucomannan and inulin.

6. The pharmaceutical composition according to claim 4 or 5, wherein the patient with chronic sinusitis is a patient with eosinophilic sinusitis or non-eosinophilic sinusitis.

7. The pharmaceutical composition according to any one of claims 4 to 6, wherein the patient with chronic sinusitis is a patient with eosinophilic sinusitis.

8. An intranasal formulation comprising the pharmaceutical composition according to any one of claims 4 to 7.

[Figure 1]

[Figure 2]

[Figure 3]

Saline

0.003 µg/mL PPS

**INTERNATIONAL SEARCH REPORT**

International application No.

PCT/JP2020/035438

**A. CLASSIFICATION OF SUBJECT MATTER**
A61K 31/726(2006.01)i; A61P 11/02(2006.01)i
FI: A61K31/726; A61P11/02

According to International Patent Classification (IPC) or to both national classification and IPC

**B. FIELDS SEARCHED**

Minimum documentation searched (classification system followed by classification symbols)
A61K31/726; A61P11/02

Documentation searched other than minimum documentation to the extent that such documents are included in the fields searched
    Published examined utility model applications of Japan     1922–1996
    Published unexamined utility model applications of Japan     1971–2020
    Registered utility model specifications of Japan     1996–2020
    Published registered utility model applications of Japan     1994–2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | SAKAI, Shinobu et al., "Chondroitin Sulfate Intake Inhibits the IgE-mediated Allergic Response by Down-regulating Th2 Responses in Mice", J Biol Chem., 2006, vol. 281(29), pp. 19872–19880, abstract | 1–8 |
| Y | SANDEN, Caroline et al., "Broad Th2 neutralization and anti-inflammatory action of pentosan polysulfate sodium in experimental allergic rhinitis", Immun Inflamm Dis., 2017, vol. 5(3), pp. 300–309, abstract | 1–8 |

☒   Further documents are listed in the continuation of Box C.       ☒   See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05 November 2020 (05.11.2020) | 24 November 2020 (24.11.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2020/035438

C (Continuation).   DOCUMENTS CONSIDERED TO BE RELEVANT

| Category* | Citation of document, with indication, where appropriate, of the relevant passages | Relevant to claim No. |
|---|---|---|
| Y | 大西伸和ほか，NC/Nga マウスの掻破行動及び皮膚局所 Th2 応答に対する微粉砕コンニャクグルコマンナンの抑制効果，アレルギー，vol. 55 no. 8/9, 2006, p. 1124, upper left column, non-official translation (OHNISHI, Nobukazu et al., "Suppressive effect of fine grinding konjac glucomannan on scratching behavior and local cutaneous Th2 response in NC/Nga mice", Japanese Journal of Allergology) | 1-8 |
| Y | TAKABAYASHI, Tetsuji et al., "Excessive Fibrin Deposition in Nasal Polyps Caused by Fibrinolytic Impairment through Reduction of Tissue Plasminogen Activator Expression", Am J Respir Grit Care Med., 2013, vol. 187(1), pp. 49-57, abstract, p. 49, right column, lines 7-12, p. 56, left column, lines 34-64, fig. 7 | 1-8 |
| Y | 藤枝重治ほか，疾患研究から次世代の治療に III. アレルギー性鼻炎 2. 好酸球性副鼻腔炎，実験医学，vol. 37, no. 10, 15 June 2019, pp. 1697-1704, pp. 1697-1698, non-official translation (FUJIEDA, Shigeharu et al., "From disease research to next-generation treatment, III. Allergic rhinitis, 2. Eosinophilic sinusitis", Experimental Medicine) | 6-7 |
| A | JP 2009-503090 A (MORRIA BIOPHARMACEUTICALS) 29 January 2009 (2009-01-29) paragraphs [0074]-[0075] | 1-8 |
| A | JP 2014-534214 A (YISSUM RESEARCH DEVELOPMENT COMPANY OF THE HEBREW UNIVERSITY OF JERUSALEM LTD.) 18 December 2014 (2014-12-18) paragraphs [0044]-[0045] | 1-8 |
| A | JP 2013-521300 A (NEOCUTIS SA) 10 June 2013 (2013-06-10) paragraph [0020] | 1-8 |

Form PCT/ISA/210 (continuation of second sheet) (January 2015)

| Patent Documents referred in the Report | Publication Date | Patent Family | Publication Date |
|---|---|---|---|
| JP 2009-503090 A | 29 Jan. 2009 | US 2007/0185052 A1 paragraphs [0031]-[0032] CN 101282733 A KR 10-2008-0065269 A | |
| JP 2014-534214 A | 18 Dec. 2014 | US 2006/0293276 A1 paragraphs [0117]-[0118] CN 103930116 A EP 2845596 A2 | |
| JP 2013-521300 A | 10 Jun. 2013 | US 2011/0217249 A1 paragraph [0021] CN 102985091 A KR 10-2013-0018739 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6463351 B
- JP H09104636 A
- JP H10203988 A
- JP H07062001 A

**Non-patent literature cited in the description**

- *Kawasaki Medical School Journal,* 2009, vol. 35 (1), 39-50
- *Japan Otolaryngology Society,* 2018, vol. 121, 1118-1120
- *Indian Journal of Otolaryngology and Head & Neck Surgery,* September 2015, vol. 67 (3), 299-307
- *Otolaryngology Immune Allergy,* 2011, vol. 29 (3), 221-227
- *Allergology International,* 2017, vol. 66, 594-602
- *Bulletin of the Japan Otolaryngology Society,* 2018, vol. 121, 1118-1120